# EUROPÄISCHE PATENTSCHRIFT

(11) **EP 0 987 063 B1**
(45) Veröffentlichungstag und Bekanntmachung des Hinweises auf die Patenterteilung: **06.05.2004**
(21) Anmeldenummer: 99117937.5
(22) Anmeldetag: 14.09.1999
(51) Int. Cl.: B09C 1/00, G01M 3/00

(54) **Verfahren zur Feststellung von Erdreichkontamination unter einer Dichtungsbahn**
Method for detecting soil contamination under a sealing liner
Procédé pour la détection d' une contamination du sol sous une bande d' étanchéité

(30) Priorität: 14.09.1998 DE 19842012
(43) Veröffentlichungstag der Anmeldung: 22.03.2000
(73) Patentinhaber: ABG Abdichtungen Boden- und Gewässerschutz GmbH, 21037 Hamburg (DE)
(72) Erfinder: ABG Abdichtungen Boden- und Gewässerschutz GmbH, 21037 Hamburg (DE)

(56) Entgegenhaltungen:
- DE-A- 4 131 085
- DE-U- 29 605 856
- US-A- 5 215 409

## Beschreibung

Die Erfindung betrifft ein Verfahren zur Feststellung der Art, des Umfangs und der Lage einer entstandenen Kontamination im Erdreich unter einer Dichtungsbahn.

Derartige überwachbare Anordnungen sind bereits bekannt und vom Anmelder mit Gebrauchsmuster DE 296 05 856 U1 am 29.03.1996 angemeldet worden. Mit diesem Gebrauchsmuster läßt sich zwar eine Kontrolle der Abdichtungsmaßnahme insbesondere an Tankstellen durchführen. Es gibt jedoch insbesondere bei Umbaumaßnahmen irritierende Ergebnisse, z.B. durch noch vorhandene Altlasten, ausgasende Domschächte bei Betankungsvorgängen u.ä., die eine zuverlässige Aussage über die Art der Meldung und damit der zuverlässigen Gewässerschutzmaßnahme nicht zulassen.

Es besteht ein Bedürfnis dahingehend, daß die mit der Anordnung des vorgenannten Gebrauchsmusters gemessenen Werte auch dann eine zuverlässige Aussage über die Gewässerschutzmaßnahme zulassen, wenn störende Fremdeinflüsse vorhanden sind.

Erfindungsgemäß wird dieses Bedürfnis durch das im Anspruch 1 angegebene Verfahren gelöst. Vorteilhafte weitere Ausgestaltungen des Verfahrens ergeben sich aus den Unteransprüchen.

Dabei ist die Luftpumpe so ausgerüstet, daß das Absaugrohr an der Ausblasöffnung angeschlossen werden kann. Diese wiederum wird mit der Gasprobennehmerabsaugstelle verbunden. Ferner ist offenbart, daß die Luftpumpe mit einem Absperrventil an ihrer Ansaugöffnung versehen ist.

Ferner kann die Absaugstelle der Prüfeinrichtung mit einem Aufsatzrohr versehen werden, dessen Durchmesser so dimensioniert ist, daß es bevorzugt dem Durchmesser des Prüfrohres entspricht, um keine Stauwirkung im Prüfrohr zu erzeugen.

In diesem Prüfrohr wird dann das Meßrohr eines Analysegerätes eingesetzt.

Durch diese Anordnung wird nun an der Ansaugstelle der Luftpumpe Frischluft angesaugt und über die Ausblasöffnung der Luftpumpe, einer Schlauchverbindung und einer Gasprobennehmer-Absaugstelle in das Prüfrohr gedrückt.

Vor Beginn des Meßvorgangs wird das an der Pumpe angeordnete Ventil geschlossen. Nach dem Anlaufen der Pumpe bis auf volle Leistung wird das Ventil geöffnet und gleichzeitig der Meßvorgang am Meßgerät gestartet.

Aufgrund der Erstmessung nach dem im vorgenannten Gebrauchsmusters beschriebenen Weise ist die voraussichtliche Dauer des Meßvorgangs bekannt. Diese Meßdauer dient auch als Richtlinie für die Dauer der nun durchzuführenden Messung. Bevorzugt wird nach Ablauf dieser Zeit ohne die Pumpe abzuschalten oder das Ventil zu schließen, in die Ansaugöffnung der Pumpe ein Prüfgas eingesprüht oder ein mit Prüfflüssigkeit getränkter Lappen gehalten.

Gemäß einer bevorzugten Anordnung muß das Meßgerät in der Lage sein, die Meßwerte in Sekundenabständen oder länger aufzuzeichnen.

Das Meßgerät muß weiterhin mit einem PC verbunden werden können, dessen Software mit dem Meßgerät kompatibel ist.

Das Meßgerät muß weiterhin in der Lage sein, die Ergebnisse der Messung auf dem PC zu übertragen.

Desweiteren muß die Software so gestaltet sein, daß das Meßergebnis grafisch dargestellt werden kann.

Wird bei einer Messung über das im vorgenannten Gebrauchsmuster beschriebene Verfahren überhöhte Meßwerte festgestellt, ist gemäß der Erfindung vor einem neuen weiteren Meßvorgang das Meßgerät so einzustellen, dass keine

Aufzeichnung vorgenommen wird, sondern lediglich die Meßwerte angezeigt werden. Nun wird die Luftpumpe gemäß Schritt b) der vorliegenden Erfindung angeschaltet und der Pumpvorgang solange durchgeführt, bis die Anzeige auf dem Meßgerät sich weitestgehend eingependelt hat und keine großen Ausschläge mehr zu verzeichnen sind. Danach wird die Luftpumpe abgeschaltet.

Nach einer bevorzugten Wartezeit von wenigstens 5 Minuten wird die Meßeinrichtung auf "Aufzeichnung" umgestellt und der Meßvorgang gestartet und die Werte dabei aufgezeichnet.

Die grafische Darstellung der Aufzeichnung zeigt im wesentlichen 3 verschiedene Variationsmöglichkeiten:
**Variante 1** Hügelartiger Verlauf (Beispiel 1 Gegenprobe)
**Variante 2** Spitzer Verlauf (kegelförmig) mit hohen Werten Beispiel 3 Gegenprobe
**Variante 3** Spitzer Verlauf (kegelförmig) mit niedrigem Wert Beispiel 2 Gegenprobe

Zur Beurteilung des Diagramms, das nach dieser erfindung entstanden ist, ist das Diagramm der Auswertung des vorgenannten Gebrauchsmusters (siehe z.B. Beispiel 1 - 4 Erstmessung) zu Vergleichszwecken heranzuziehen. Zu beachten ist dabei, daß sofern die Anschlüsse der Rohrverbindung an den Probeentnahmestellen nicht getauscht werden, die Auswertung spiegelbildlich dargestellt ist.

Sind die Werte dieser Messung gegenüber der Erstmessung stark reduziert (z.B. 20 % unterhalb der Erstmessung), so weist dies auf gasförmig vorhandene Partikel hin, von denen keine Gefahr für Boden und Grundwasser ausgeht. Außerdem ist auch eine Schadstelle an der Abdichtung auszuschließen.

Eine nur geringfügig reduzierte Messung weist auf eine sogen. Naßkontamination hin, die mehrere Ursachen haben kann: Mängel an der tanktechnischen Anlage wie Rohrleitungen, Domschächten, Zapfsäulen usw. Aber auch eine mangelhafte Abdichtung kann hier die Ursache sein. Da eine akute Wassergefährdung anzunehmen ist, sind Maßnahmen zur Feststellung und Beseitigung einzuleiten.

Ein hügelartiges Diagramm weist immer auf eine großflächige Kontaminationsquelle hin, wie sie z.B. bei Altlasten festzustellen ist. Eine schadhafte Abdichtung, eine Leckage in einem Rohr, aber auch ein undichter Domschacht verursachen nur punktuelle Schadstellen, die ein kegelartiges Diagrammbild ergeben.

### Technische Voraussetzungen

Bekanntes gelochtes Prüfrohrsystem mit den beiden offenen Enden, beieinander liegend, mengenlimitierte Luftpumpe, PID- oder FID-Meßgerät mit Aufzeichnungsmöglichkeit und Software für die Darstellung von Tabellen und Diagrammen.

### Erfindung

Umbau der vorgenannten Pumpe, so daß der Verbindungsschlauch zwischen Absaugelement der Prüfleitung und der Ansaugstelle der Pumpe an die Ausblasöffnung der Pumpe direkt angekoppelt werden kann.

Am Ansaugelement der Prüfleitung wird ein ca. 50 cm langes Verlängerungsstück gasdicht angekoppelt. In dieses Verlängerungsstück wird die Sonde des PID- oder FID-Meßgerätes eingesetzt.

Wie bekannt, werden gasförmige Partikel über ein gelochtes Prüfrohr unterhalb einer Abdichtung mit einer mengenlimitierten Pumpe angesaugt und mit dem PhD- oder FID-Meßgerät in kurzen Schritten gemessen und aufgezeichnet.

Das Meßgerät ist einzustellen auf zeitlich eng begrenzte Abschnitte z. B. 1 od. 2 Sekunden-Abschnitten. Die Meßdauer sollte dem gleichen Rhytmus entsprechen um eine ununterbrochene Aufzeichnung zu erhalten.

Für den Fall, daß überhöhte Werte gemessen werden, ist zur Feststellung der Art der Kontamination und Zuordnung auf eine aktuelle Kontaminationsquelle folgendes Verfahren anzuwenden.
1. **Vorbereitung**
   Die Verbindungsleitung zwischen Absaugelement des Prüfrohres und Ansaugelement der Luftpumpe wird auf die Ausblasöffnung der Luftpumpe umgesetzt.
2. **Luftpumpe**
   Die Sonde des Meßgerätes wird in das Verlängerungsrohr der Ansaugstelle des Prüfrohres eingesetzt, jedoch so, daß die ausströmende Luft seitlich an der Sonde des Meßgerätes ungehindert ausströmen kann.
3. **Reinigungsvorgang**
   Nun wird die Luftpumpe mit geöffnetem Ventil gestartet und das Meßgerät ohne Aufzeichnung in Betrieb genommen. Dieser Reinigungsvorgang wird solange durchgeführt, bis sich die angezeigten Werte nicht mehr nach unten korrigieren und sich in relativ konstantem Rahmen bewegen. Nun werden Pumpen und Meßgeräte wieder abgeschaltet.
4. **Meßvorgang**
   Nach einer gewissen Erholungsfrist von beispielsweise 5 Minuten wird das Ventil der Luftpumpe geschlossen und das Meßgerät mit Modus "Aufzeichnen" gestartet. Der Öffnungszeitpunkt des Ventils an der Luftpumpe wird dokumentiert. Der Meßvorgang wird solange durchgeführt wie die Erstmessung (mit saugen) gedauert hat, zzgl. einiger Meßpunkte als Sicherheitsreserve.
5. **Feststellung der Prüfdauer im Prüfrohr**
   Die genaue Länge der Meßdauer wird dadurch ermittelt, daß zu einem dokumentierten Zeitpunkt ein kurzer Testgasstoß in die Ansaugluft der Luftpumpe gegeben wird. Nach Überschreiten des Höchstpunktes, das durch das Testgas ausgelöst wurde, ist der Meßvorgang beendet.
6. **Auswertung**
   Nach der Ubertragung der Daten vom Meßgerät auf den PC werden alle gesammelten Daten komplett ausgedruckt. Anhand der Feststellung der Laufdauer der Messung mit dem Prüfgas wird die zuvor durchgeführte Messung exakt auf die Länge der Prüfgasmessung gekürzt und diese Werte auf ein separates Meßprotokoll übertragen.
   Die einzelnen Meßpunkte sollten auf dem Meßprotokoll folgende Angaben enthalten.

Laufende Nummer
Datum der Aufzeichnung
Zeitpunkt der Aufzeichnung (einschl. Sekunden)
Festgestellter Meßwert, bevorzugt in ppm

Dieses Meßprotokoll ist dann auch noch grafisch auf einem Meßdiagramm darzustellen, wobei auf der unteren Linie die Gesamtzahl der einzelnen Meßabschnitte und auf der senkrechten Linie eine Tabelle für die Höhe der Werte der Einzelmeßpunkte dargestellt sind.

Die Auswertung und das Diagramm der neuen Beprobung gemäß der Erfindung, die als "Gegenprobe" bezeichnet wird, ist gegenüber der Erstmessung nach dem bekannten Verfahren spiegelbildlich.

### 7. Ergebnis der Beprobung

### Beispiel 1

*Erstmessung* (Liste 1-1.wks)
typisches Diagramm für eine Altlast, da hügelartiges Diagramm
Höchstmessung 240 ppm
*Gegenprobe -* (Liste 1-2.wks)
gleichartiges Diagramm wie Erstmessung, jedoch Höchstmessung nur 25 ppm (ca. 10% der Erstmessung)

*Bewertung:* keine aktuelle Wassergefährdung

### Beispiel 2

*Erstmessung* (Liste 2-1.wks)
typisches Diagramm für eine punktuelle Kontamination - Höchstmessung 85 ppm
*Gegenprobe* (Liste 2-2.wks)
gleichartiges Diagramm jedoch mit einer Höchstmessung von 9 ppm (ca. 10% der Erstmessung)
*Bewertung:* keine aktuelle Wassergefährdung

### Beispiel 3

*Erstmessung* (Liste 3-1.wks)
typisches Beispiel einer punktuellen Meldestelle mit Höchstwert 535 ppm
*Gegenprobe* (Liste 3-2.wks)
gleichartiges Diagramm jedoch mit Höchstwert - 320 ppm (ca. 60% der Erstmessung)
*Bewertung:* Nasskontamination mit akuter Wassergefährdung - Maßnahmen zur Schadensfeststellung und -beseitigung sind einzuleiten.

### Beispiel 4

*Erstmessung* (Liste 4-1.wks)
typisches Beispiel einer Altlast mit Höchstwert von 360 ppm
*Gegenprobe* (Liste 4-2.wks)
deutlich hebt sich aus der nur noch in geringen Mengen sichtbaren Altlast eine aktuellen Kontamination hervor
*Bewertung:* Nasskontamination mit aktueller Wassergefährdung - Maßnahmen zur Schadensfeststellung und
-beseitigung sind einzuleiten.

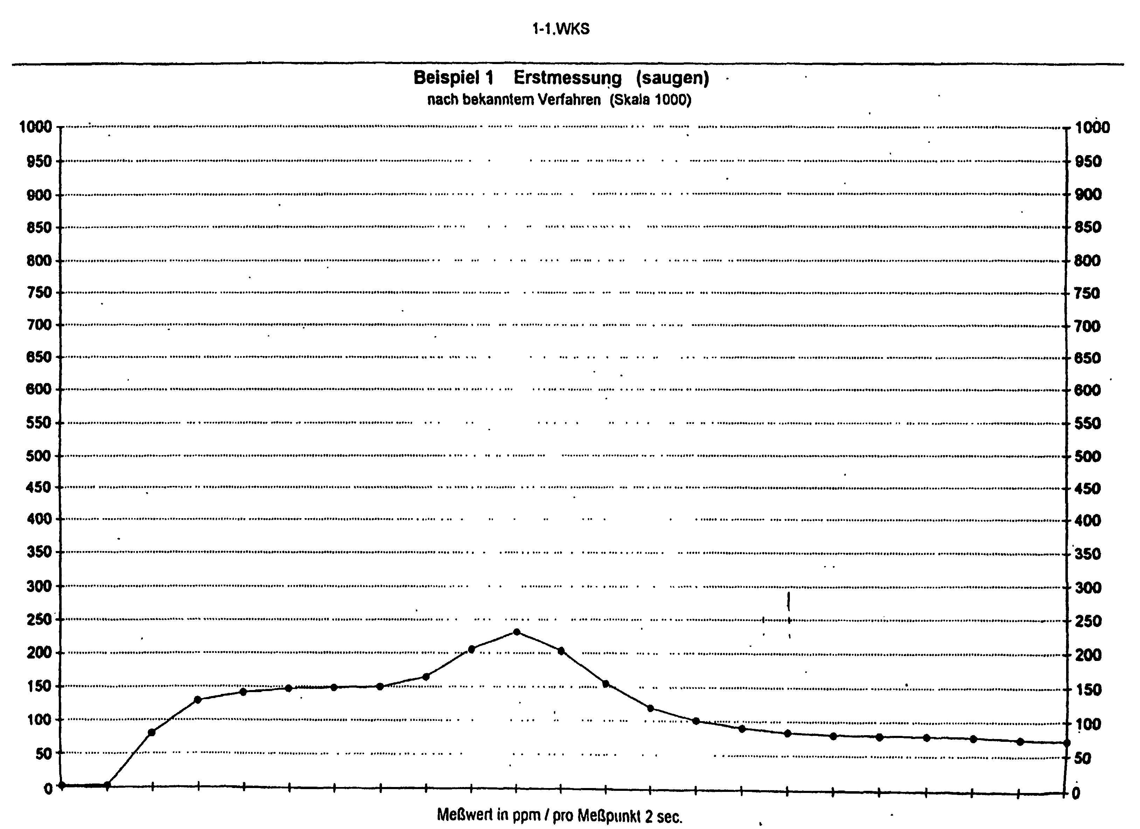

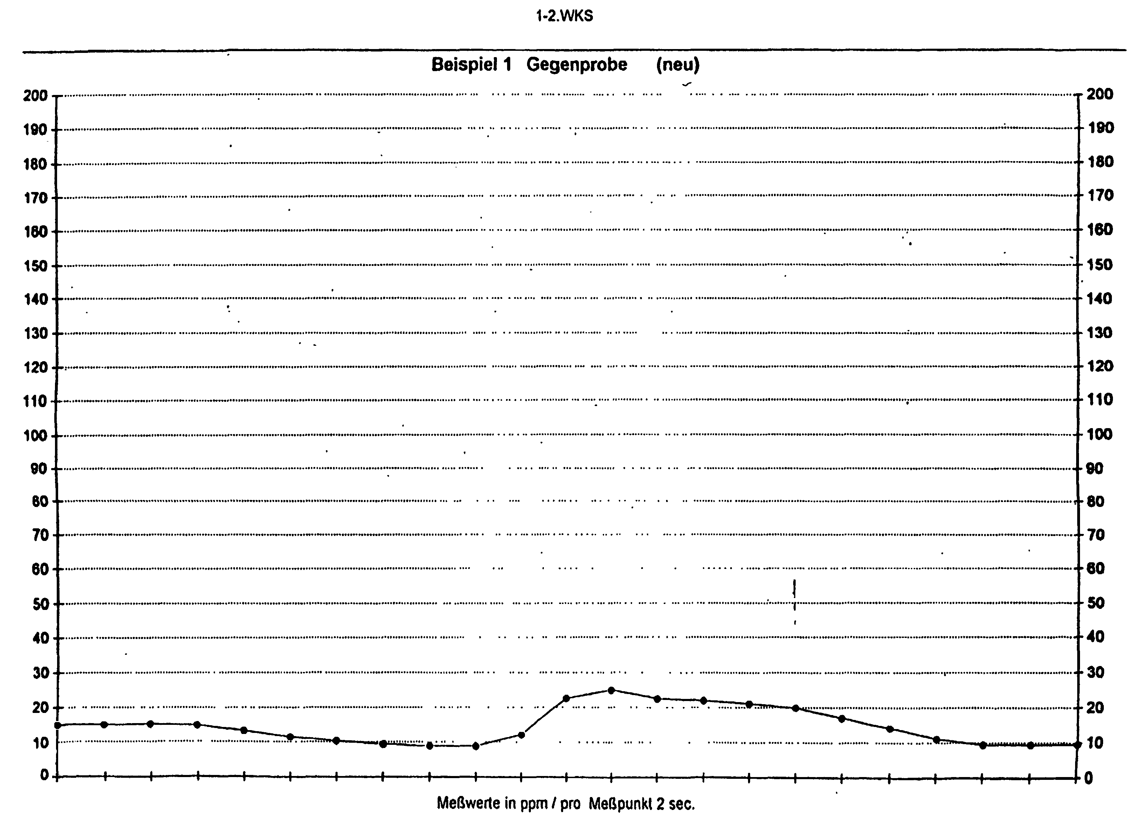

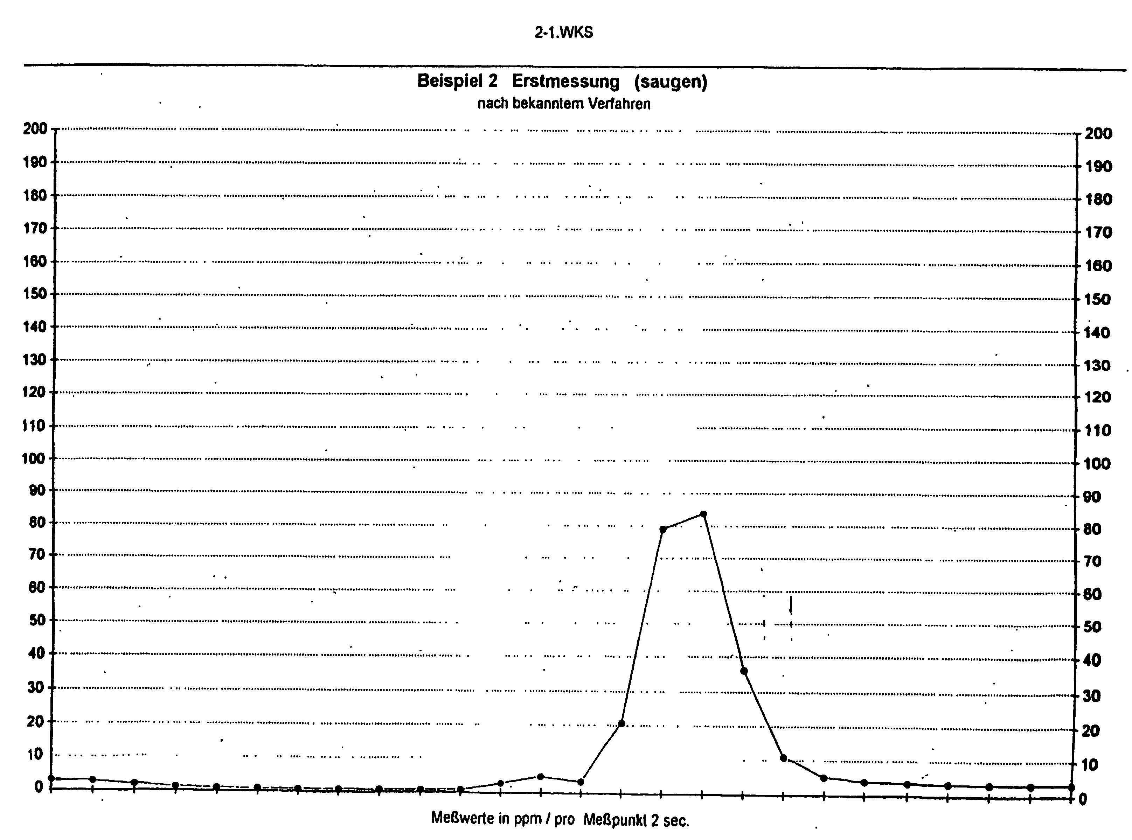

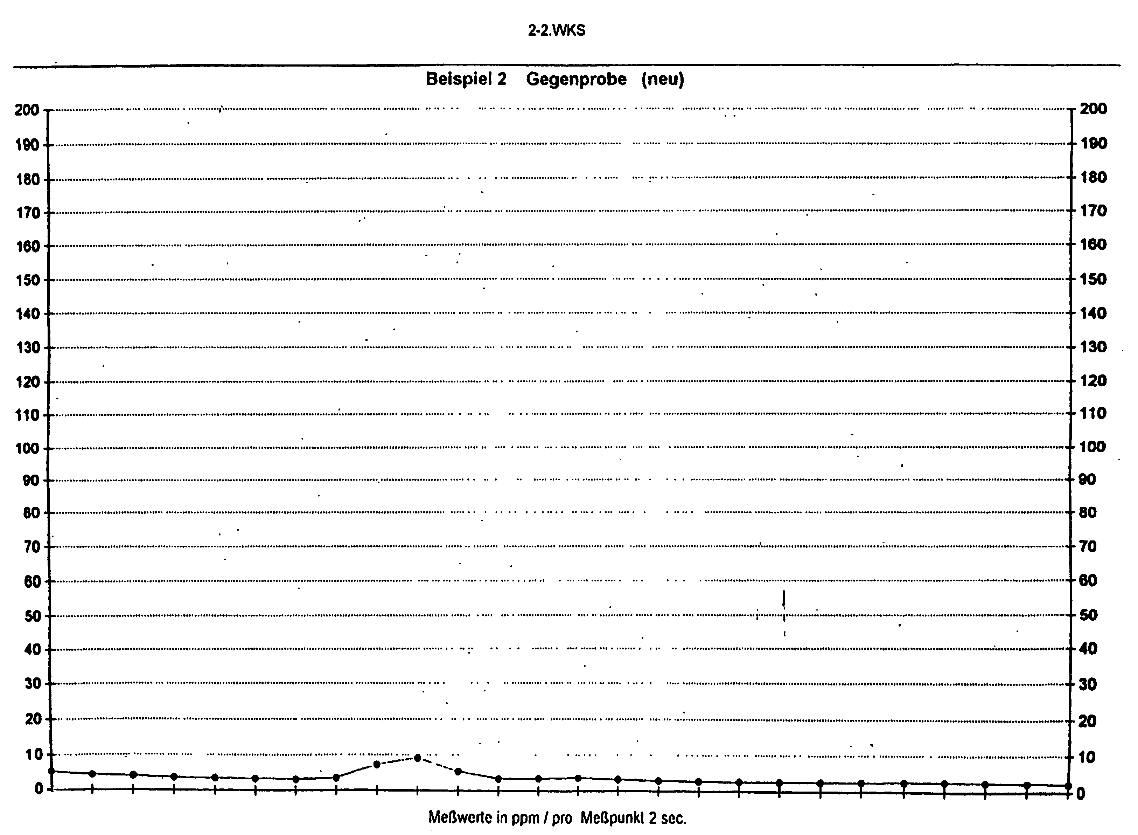

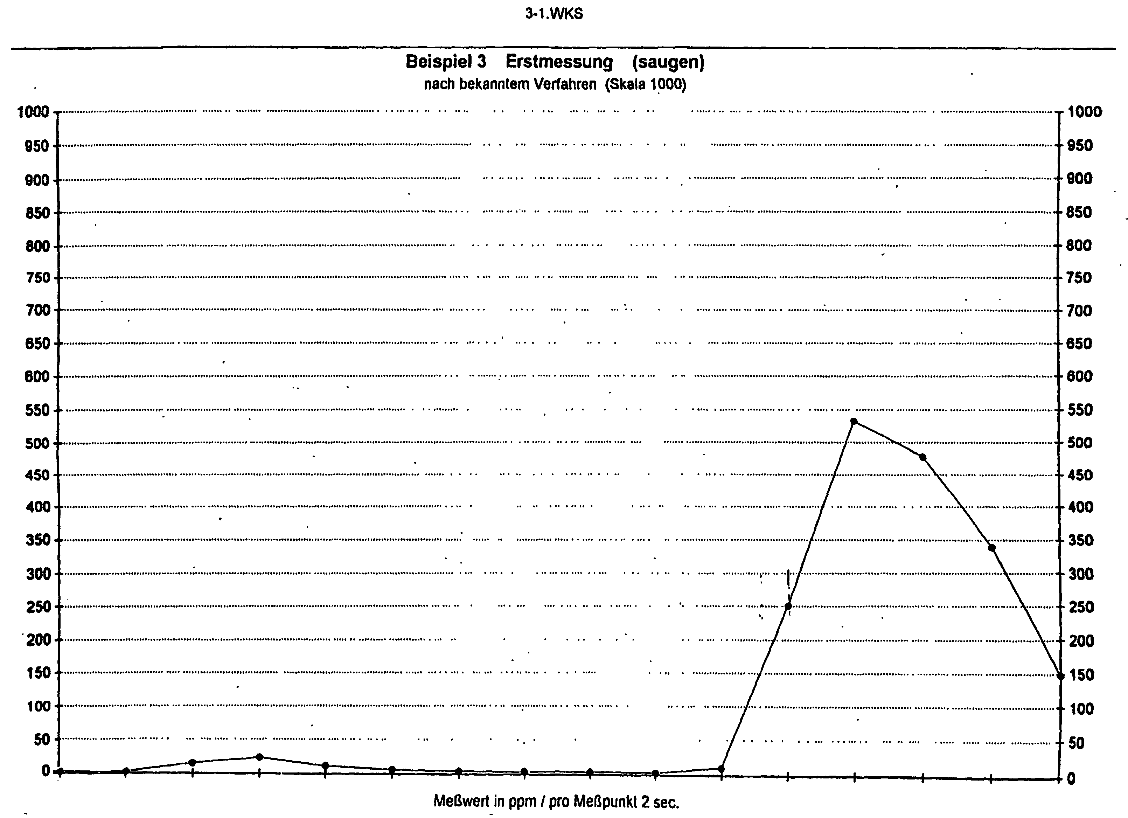

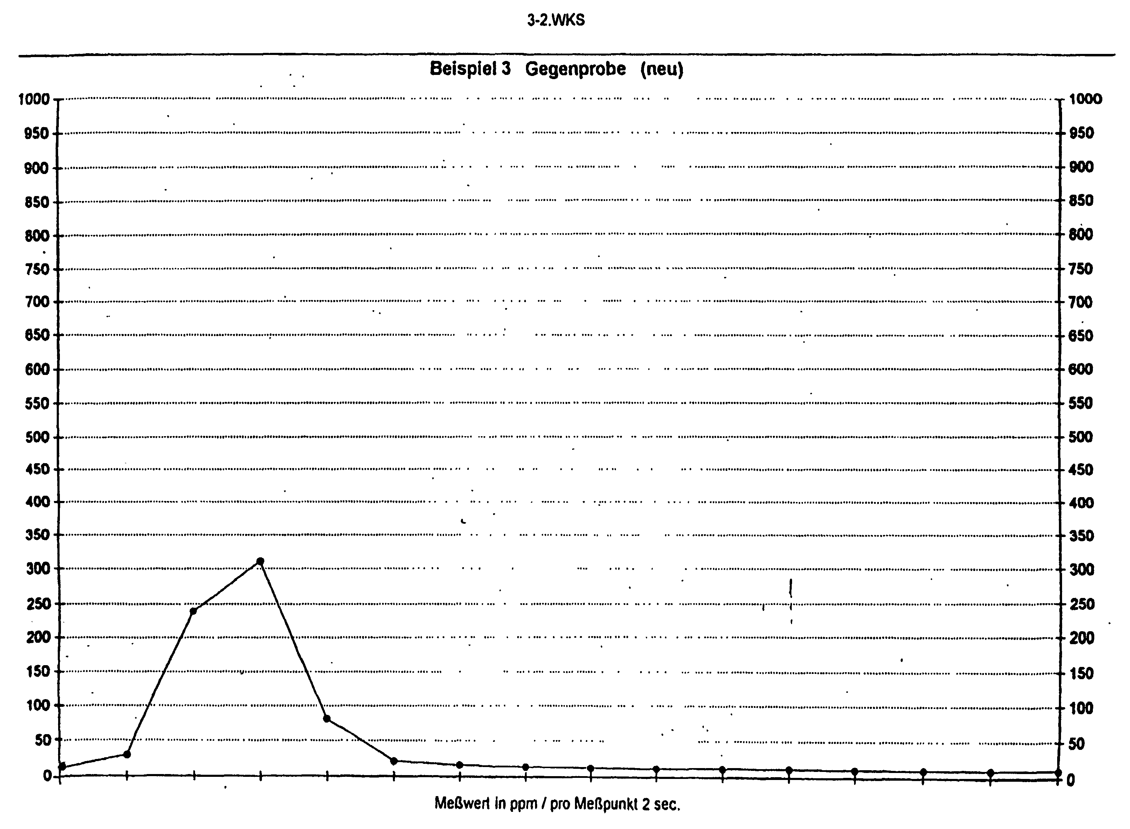

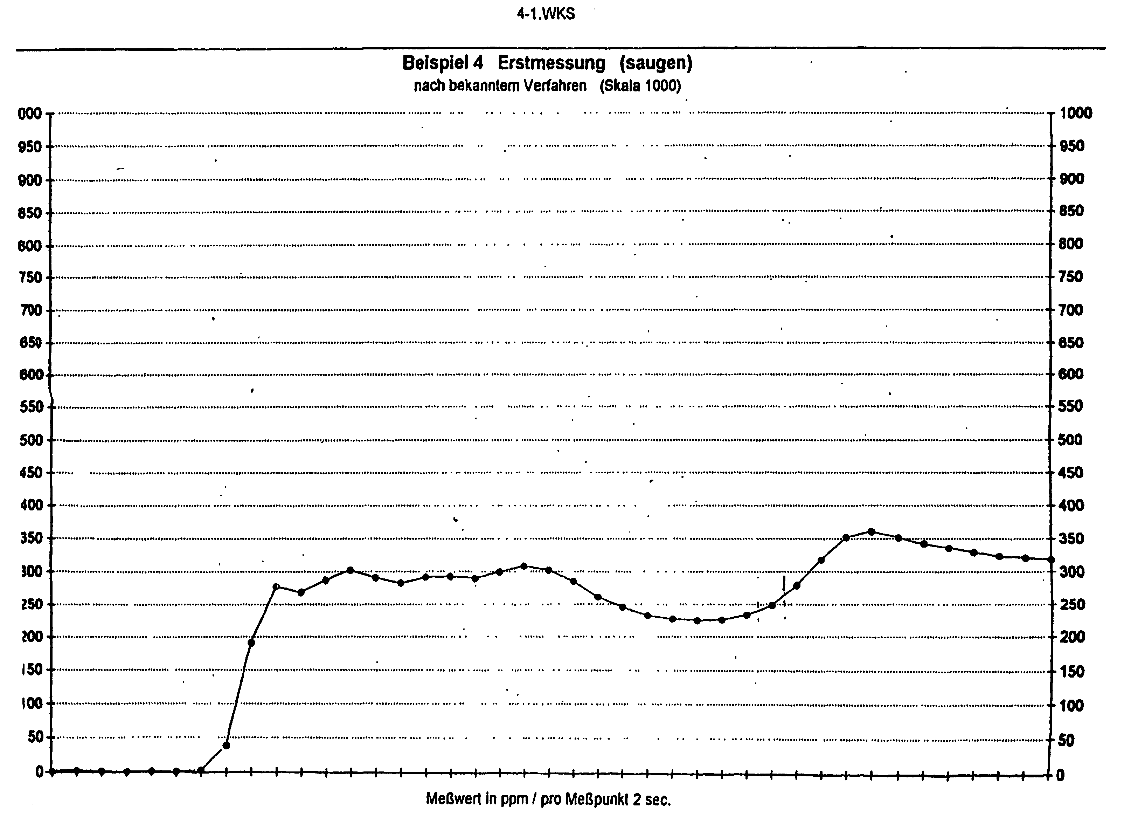

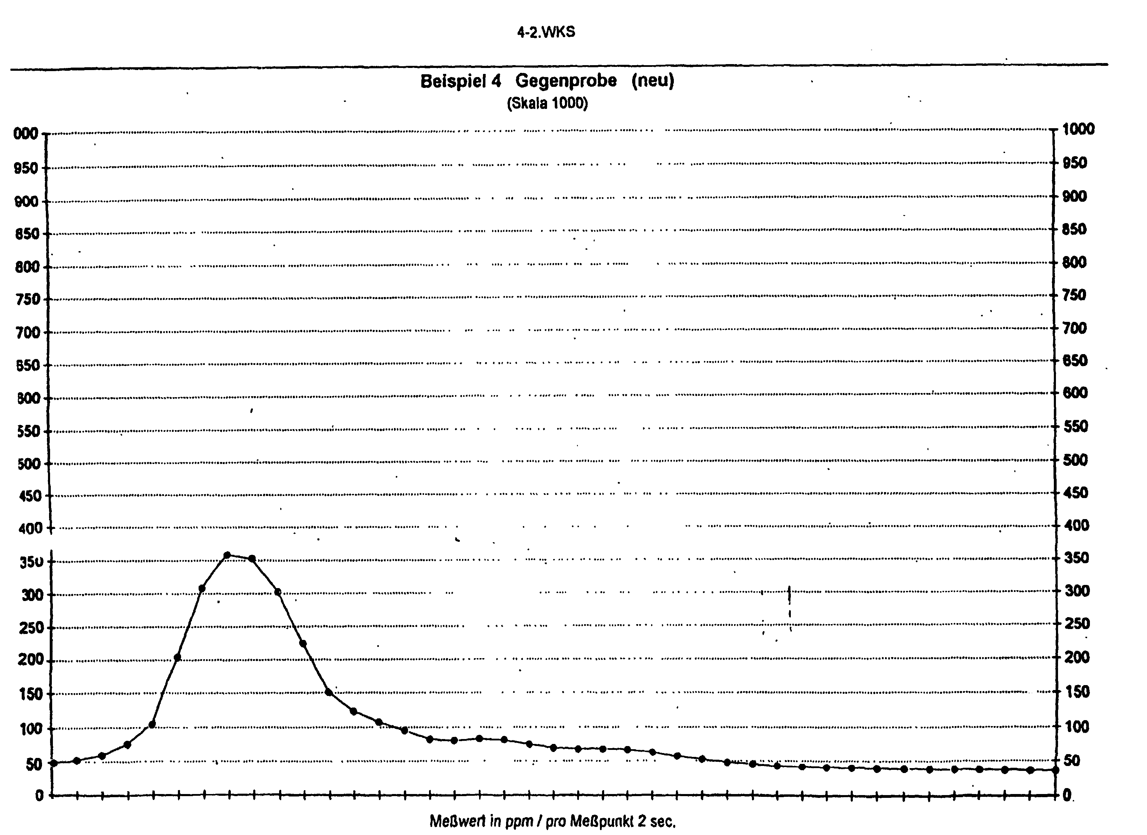

## Patentansprüche

1. Verfahren zur Feststellung der Art, des Umfangs und der Lage einer entstandenen Kontamination im Erdreich unter einer Dichtungsbahn mittels eines Systems aus einem dort angeordneten gelochten Prüfrohr, dessen offene Enden nebeneinanderliegend mit einer mit einem Absaug-Ventil verschließbaren mengenlimitierten Luftpumpe verbindbar sind, und aus einem PID- oder FID-Meßgerät mit Aufzeichnungsmöglichkeit und Software für die Darstellung von Tabellen und Diagrammen, bestehend aus folgenden Verfahrensschritten:
a) Gasförmige Partikel werden über das Prüfrohr an der Absaugseite des Prüfrohres abgesaugt und zeitbezogen mit dem PID- oder FID-Meßgerät in kurzen Schritten gemessen und aufgezeichnet (Beispiel 1, 2, 3, 4 - Erstmessung);
b) falls beim Schritt a) überhöhte Werte gemessen werden, wird die Prüfrohrabsaugseite mit Ausblasluft der Luftpumpe beaufschlagt, wobei an der Ansaugstelle der Luftpumpe Frischluft angesaugt wird und eine Meßsonde des PID- oder FID-Meßgerätes in einen Rohrbereich vor der Prüfrohransaugöffnung eingeführt;
c) die Luftpumpe wird mit offenem Absaugventil ohne Messung zur Reinigung betrieben, bis die Meßwerte in einem konstanten Rahmen liegen;
d) der Betrieb der Luftpumpe wird während einer gewissen Erholungsfrist bzw. Ruhephase von 3 bis 5 Minuten unterbrochen;
e) Beginn des Meßvorgangs mit Ausblasluft der Luftpumpe beaufschlagter Prüfrohr absaugseite und mit zunächst geschlossenem Absaug-Ventil und anschließend Durchführung einer zeitbezogenen Meßdatenerfassung und -dokumentation annähernd über die gleiche Dauer wie die Saugmessung gemäß Schritt (a) (Beispiel 1, 2, 3, 4 - Gegenprobe);
f) Vergleich der Meßergebnisse gemäß Schritt e) mit denen gemäß Schritt a), wobei eine vergleichsweise starke Wertreduzierung beim Schritt e) gegenüber Schritt a) auf eine gefahrlose Kontamination mit gasförmigen Partikeln und eine beim Schritt e) nur geringfügig reduzierte Messung gegenüber Schritt a) auf eine zu beseitigende sogenannte Naßkontamination hinweist; und
g) Bestimmen der Lage der Kontamination im Bereich eines Prüfrohrabschnitts über die Zeitmessung.

2. Verfahren nach Anspruch 1, **dadurch gekennzeichnet, daß** die Messung gemäß Schritt a) und Schritt e) in gegenseitig eng begrenzten Abschnitten von vorzugsweise 1 bzw. 2 Sekunden durchgeführt werden, wobei die Meßdauer zum Erhalt einer ununterbrochenen Aufzeichnung dem gleichen Rhytmus entspricht.

3. Verfahren nach Anspruch 1 oder 2, **dadurch gekennzeichnet, daß** die genaue Länge der Meßdauer beim Schritt e) dadurch ermittelt wird, daß zu einem dokumentierten Zeitpunkt ein kurzer Testgasstoß in die Ansaugluft der Luftpumpe gegeben wird, wobei nach Überschreiten des durch das Testgas ausgelösten Meßhöchstpunktes der Meßvorgang beendet wird.

## Claims

1. Procedure for assessing the type, scope and location of soil contamination under a plastic sheeting through a system consisting of a perforated test pipe placed there, whose adjacent open ends can be linked with an operable and a purge valve volume-controlled air pump, and a PID or FID measuring device with recording option and software for chart and diagram display, consisting of following process steps:
a) gaseous particles are sucked off via the test pipe on the suction side of the test pipe and measured and recorded with time orientation by the PID or FID measuring device with short intervals, (i.e. 1,2,3,4 - initial measurement);
b) in the event excessive values are measured during the first step a), blow-off air supplied from the air pump is admitted to the test pipe suction side - wherefore fresh air is induced into the intake side of the air pump - and a measuring probe of the PID or FID measuring device is introduced in a pipe section upstream the test pipe intake;
c) The air pump is operated for cleaning purposes with open purge valve without measurement until the measured values remain in a constant range;
d) The air pump operation is interrupted for a defined recuperative interval, that is to say a rest period of 3 to 5 minutes;
e) Measurement starts first with blow-off air from the air pump induced into the test pipe suction side and with closed purge valve, followed by a time-oriented measurement data acquisition and data logging that lasts approx. the same time as the suction measurement according to step (a) (i.e. 1,2,3,4 - crosscheck);
f) Comparison of the measurement results according to step e) with the values according to step a), where a comparatively strong reduction of value with step e) compared to step a) indicates a safe contamination with gaseous particles whereas a during step e) only slightly reduced measurement compared to step a) indicates a so-called wet contamination that must be removed; and
g) Assessment of contamination location within a test pipe section via chronometry

2. Procedure according to claim 1, **characterised in that** the measurements are carried out according to step a) and step e) with reciprocally closely limited intervals of preferably 1 or 2 seconds, where the measuring period has the same rhythm so that continuous recording is ensured.

3. Procedure according to claim 1 or 2, **characterised in that** the exact duration of the measuring period during step e) is assessed through a short test gas pulse induced into the intake air of the air pump at a documented time, in which measurement is terminated when the upper measuring limit triggered by the test gas is exceeded.

## Revendications

1. Procédé permettant de déterminer la nature, l'étendue et la position d'une contamination du sol sous une bande d'étanchéité à l'aide d'un système qui consiste d'un tuyau à essai perforé placé là-bas, dont les extrémités ouvertes adjacentes peuvent être conjugués avec une pompe à air pouvant être fermée et le débit limité à l'aide d'une soupape d'aspiration, ainsi que d'un appareil de mesure PID ou FID doté d'option d'enregistrement et d'un logiciel permettant l'affichage de tableaux et de diagrammes, comprenant les séquences de procédure suivantes :
a) des particules gazeuses sont aspirées à travers le tuyau à essai - du côté d'aspiration du tuyau à essai - et mesurées et enregistrées à l'aide d'un appareil de mesure PID ou FID en fonction du temps avec des intervalles courts (par ex. 1,2,3,4 - mesure initiale) ;
b) le côté aspiration du tuyau à essai est alimenté en air de purge envoyé par la pompe à air, tandis que de l'air frai est aspiré du côté aspiration de la pompe à air lorsque des valeurs excessives sont mesurées lors de l'étape a), et une sonde de mesure de l'appareil de mesure PID ou FID est introduit dans un secteur du tuyau en amont de l'orifice d'aspiration du tuyau à essai ;
c) Pour le nettoyage, la pompe à air est opérée avec soupape d'aspiration ouverte sans qu'une mesure ait lieu jusqu'à ce que les valeurs mesurées restent dans une plage constante ;
d) Le service de la pompe à air est interrompu pour une période définie de repos, à savoir 3 à 5 minutes de phase de repos ;
e) Le repérage ou mesurage commence par l'alimentation en air de purge de la pompe à air du côté d'aspiration du tuyau à essai et avec soupape d'Aspiration d'abord fermée, suivi par la saisie de données de mesure et une documentation de celles-ci réalisées en fonction du temps durant à peu près aussi longtemps que la mesure par aspiration d'après l'étape (a) (par ex. 1,2,3,4 - contre-épreuve) ;
f) Comparaison des résultats de mesure d'après l'étape e) avec ceux d'après l'étape a), sachant qu'une réduction relativement forte de la valeur lors de l'étape e) par rapport à l'étape a) constitue une indice pour une contamination non dangereuse de particules gazeuses, tandis qu'un écart faible, voir une diminution faible de la mesure lors de l'étape e) par rapport à l'étape a) constitue une indice pour une contamination « mouillée » à éliminer ; et
g) Détermination de la position de la contamination dans un secteur, voir tronçon du tuyau à essai grâce au chronométrage.

2. Procédé d'après la revendication 1, **se caractérisant en ce que** la mesure réalisée d'après l'étape a) et l'étape e) se font dans des intervalles très courts, de préférence 1 ou 2 secondes, sachant que la durée de mesure ait le même rythme pour assurer un enregistrement continu.

3. Procédé d'après la revendication 1 ou 2, **se caractérisant en ce que** la durée exacte du mesurage lors de l'étape e) est déterminée à l'aide d'une impulsion brève de gaz à essai envoyée dans l'air d'aspiration de la pompe à air en un instant documenté, sachant que le mesurage se termine par le dépassement de la valeur limite supérieure de mesure par le gaz à essai.
